# EUROPÄISCHE PATENTSCHRIFT

(11) **EP 2 902 010 B1**
(45) Veröffentlichungstag und Bekanntmachung des Hinweises auf die Patenterteilung: **31.01.2018**
(21) Anmeldenummer: 14153833.0
(22) Anmeldetag: 04.02.2014
(51) Int. Cl.: A61K 8/60, A61K 8/46, A61Q 5/02, A61Q 9/02, A61Q 19/10, C11D 1/83, C11D 17/00, C11D 1/66, C11D 1/28

(54) **Wäßrige tensid-Zusammensetzungen**
Aqueous tenside compositions
Compositions de tensioactifs aqueuses

(43) Veröffentlichungstag der Anmeldung: 05.08.2015
(73) Patentinhaber: BASF SE, 67056 Ludwigshafen am Rhein (DE)
(72) Erfinder: Max, Eva, 40599 Düsseldorf (DE); Behler, Ansgar, 46240 Bottrop (DE)
(74) Vertreter: Reinhardt, Jürgen

(56) Entgegenhaltungen:
- EP-A2- 0 070 074
- DE-A1- 3 827 778
- DE-A1-102007 038 029
- US-A- 5 591 377
- US-A1- 2005 153 853
- US-A1- 2012 208 898

## Beschreibung

### Gebiet der Erfindung

Die vorliegende Erfindung betrifft wäßrige Tensid-Zusammensetzungen mit einem Gehalt an alpha-Sulfofettsäuredisalzen sowie speziellen Alkyl- bzw. Alkenyloligoglykosiden.

### Stand der Technik

Anionische Tenside gehören zu den am weitesten verbreiteten grenzflächenaktiven Verbindungen und werden außer in Wasch- und Reinigungsmittel auch auf dem Gebiet der Kosmetik vielfältig eingesetzt. Übliche anionische Tenside, wie sie vor allem in der Kosmetik eingesetzt werden, sind die Salze von Alkylethersulfaten (Alkylpolyethersulfate, Fettalkoholpolyglycolethersulfate, verkürzt auch Ethersulfate). Sie zeichnen sich durch starkes Schaumvermögen, hohe Reinigungskraft, geringe Härte- und Fettempfindlichkeit aus und finden vielfach Verwendung zur Herstellung von kosmetischen Produkten wie beispielsweise Haarshampoos, Schaum- oder Duschbädern, aber auch in Handgeschirrspülmitteln.

Für viele aktuelle Anwendungen werden an anionische Tenside außer einer guten grenzflächenaktiven Wirkung weitere Anforderungen gestellt. Insbesondere in der Kosmetik ist eine hohe dermatologische Verträglichkeit erforderlich. Des Weiteren ist in der Regel eine ausreichende Wasserlöslichkeit, eine gute Verträglichkeit mit möglichst vielen der in der Kosmetik eingesetzten Wirk- und Hilfsstoffen, ein gutes Schaumbildungsvermögen und eine gute Verdickbarkeit erwünscht. Des Weiteren besteht ein Bedarf an anionischen Tensiden, die zumindest teilweise aus biogenen Quellen und speziell auch nachwachsenden Rohstoffen hergestellt werden können. Weiterhin besteht auch ein Bedarf an Tensiden, die keine alkoxilierten Gruppen aufweisen und die somit insbesondere den Einsatz von Ethylenoxid zu ihrer Herstellung überflüssig machen.

DE-A-38,27,778 beschreibt pastenförmiges Wasch- und Reinigungsmittel. Dabei werden wäßrige Pasten eingesetzt, die ein Alkylglucosid und ein alpha-Sulfofettsäuredisalz enthalten. Bevorzugt ist dabei die Kombination eines C_{12/14}-Alkylglucosids mit einem alpha-Sulfofettsäuredisalz auf Basis von C_{16/18}-Fettsäure (Talgfettsäure), siehe etwa Spalte 3, Zeilen 12-19 und Beispiel 1. Als Verwendungszweck nennt das Dokument spezifisch Wasch- und Reinigungsmittel, wobei Spalte 5, Zeilen 16-22 zu entnehmen ist, das es um das manuelle Reinigen von Gegenständen mit harten Oberflächen, etwa Geschirr, und um das Waschen von Textilien mit der Hand oder in der Waschmaschine geht.

US-A- 2012/208,898 offenbart flüssige Tensid-Zusammensetzungen enthaltend ein oder mehrere alpha-Sulfofettsäuredisalze (A) sowie ein Alkylglykosid (B), wobei das Gewichtsverhältnis der Verbindungen (A) und /B) 1 : 0,5 beträgt. Beispiel 22 (vergl. Tabelle 3) dieser Anmeldung beschreibt eine Zusammensetzung, die 10% Aktivsubstanz *Alphastep PC-48* und 0,5% Aktivsubstanz *Glucopon 625 UP* enthält. Dabei handelt es sich bei *Alphastep PC-48* gemäß Tabelle 7 (vergl. Seite 7) um eine Mischung von C_{12/18}-Estersulfonat ("Monosalz") und C_{12/18}-Disalz, wobei das Verhältnis Monosalz zu Disalz 7 : 1 beträgt. Daraus ergibt sich für das Beispiel 22 ein Verhältnis von Disalz (A) zu APG (B) von 1 : 0,4.

US-A-5,591,377 offenbart viskose, auf Wasser basierende Tensid-Zusammensetzungen enthaltend Alkylglycoside und alpha-Sulfofettsäuresalzen mit einem Gewichtsverhältnis im Bereich von 40 : 1 bis 5 : 1.

### Beschreibung der Erfindung

Die Aufgabe der vorliegenden Erfindung hat darin bestanden, wäßrige Tensid-Zusammensetzungen mit einem Gehalt an Alkyl- bzw. Alkenyloligoglycosiden bereitzustellen, die sich durch ein exzellentes Schaumvermögen, insbesondere im Anschäumverhalten auszeichnen. Das Anschäumverhalten spielt bei sogenannten Rinse-off-Produkten, worunter Produkte zu verstehen sind, die beim Reinigen oder Pflegen mit der Haut in Berührung kommen, dann aber wieder abgewaschen werden (z.B. Duschgele, Duschformulierungen, Shampoos, Flüssigseifen, usw.) eine sehr wichtige Rolle. In diesem Bereich ist ein möglichst hohes Schaumvolumens erwünscht.

Die wäßrigen Tensid-Zusammensetzungen sollten darüber hinaus Hydrolysestabilität sowohl im sauren als auch im alkalischen pH-Bereich aufweisen und möglichst frei von Bestandteilen sein, die Ethylen- oder Propylenoxid-Bausteine oder Sulfatgruppen enthalten.

Die wäßrigen Tensid-Zusammensetzungen sollten außerdem entweder gut auf eine ausreichend hohe Viskosität verdickbar sein oder bereits von sich aus eine ausreichend hohe Viskosität aufweisen. Als ausreichend hohe Viskosität wird im Rahmen der vorliegenden Erfindung ein Wert von 1000 mPas oder höher und insbesondere ein Wert im Bereich von 2000 bis 8000 mPas verstanden (gemessen mit einem Brookfield RV Laborrheometer bei 20°C, 12 U/min, Spindelset RV 02 bis 07 (Spindelauswahl je nach Viskositätbereich)). "mPas" bedeutet bekanntlich Millipascalsekunden.

Die wäßrigen Tensid-Zusammensetzungen sollten ferner eine Lagerstabilität bei Raumtemperatur (23 °C) über mindestens 8 Wochen aufweisen, ohne dass irgendwelche sichtbaren Veränderungen (etwa Austrübungen, Phasentrennungen, und dergleichen) auftreten und ohne dass Viskositätsveränderungen oder Änderungen der chemischen Zusammensetzung auftreten.

Gegenstand der Erfindung sind zunächst wäßrige Tensid-Zusammensetzungen enthaltend
- ein oder mehrere alpha-Sulfofettsäuredisalze (A) der allgemeinen Formel (I),

   R¹CH(SO₃M¹)COOM² (I),

   worin der Rest R¹ einen linearen oder verzweigten Alkyl- oder Alkenylrest mit 6 bis 16 C-Atomen bedeutet und die Reste M¹ und M² - unabhängig voneinander - ausgewählt werden aus der Gruppe H, Li, Na, K, Ca/2, Mg/2, Ammonium und Alkanolamine,
- ein oder mehrere Alkylglycoside (B) der allgemeinen Formel (II),

   R²O-[G]ₚ (I)

   in der R² für einen Alkyl- und/oder Alkenylrest mit 8 bis 18 C-Atomen, G für einen Zuckerrest mit 5 oder 6 Kohlenstoffatomen und p für Zahlen zwischen 1 und 10 steht,
- **Wasser,**
   wobei folgende Maßgaben gelten:
   - In Bezug auf die Verbindungen (A) gilt, dass der Anteil der Verbindungen (A), bei denen der Rest R¹ ein Alkyl- oder Alkenylrest mit 14 oder mehr C-Atomen ist, - bezogen auf die Gesamtmenge der Verbindungen (A) in den wäßrigen Tensid-Zusammensetzungen - bei 20 Gew.-% oder weniger liegt;
   - in Bezug auf die Verbindungen (B) gilt, dass der Anteil der Verbindungen (B), bei denen der Rest R² ein Alkyl- oder Alkenylrest mit 15 oder mehr C-Atomen ist, - bezogen auf die Gesamtmenge der Verbindungen (B) in den wäßrigen Tensid-Zusammensetzungen - bei 5 Gew.-% oder weniger liegt;
   - sofern die wäßrigen Tensid-Zusammensetzungen ein oder mehrere Estersulfonate (E) der allgemeinen Formel (V),

      R⁵CH(SO₃M⁵)COOR⁶ (V)

      worin der Rest R⁵ einen linearen oder verzweigten Alkyl- oder Alkenylrest mit 6 bis 18 C-Atomen bedeutet und der Rest R⁶ einen linearen oder verzweigten Alkyl- oder Alkenylrest mit 1 bis 20 C-Atomen bedeutet, wobei der Rest R⁶ logischerweise erst ab 3 C-Atomen ein Alkenylrest sein oder verzweigt sein kann, und der Rest M⁵ ausgewählt wird aus der Gruppe Li, Na, K, Ca/2, Mg/2, Ammonium und Alkanolamine, enthält, gilt, dass die Verbindungen (A) - bezogen auf die Gesamtheit der Verbindungen (A) und (E) - zu 50 Gew.-% oder mehr - und insbesondere zu 90 Gew.-% oder mehr - vorliegen müssen;
   - das Gewichtsverhältnis der Verbindungen (A) : (B) in den wäßrigen Tensid-Zusammensetzungen liegt im Bereich von 0,6 : 1 bis 1 : 0,6.

### Zu den Verbindungen (A)

Die Verbindungen (A), die im Rahmen der vorliegenden Erfindung als **alpha-Sulfofettsäuredisalze** bezeichnet werden, sind für die erfindungsgemäßen wäßrigen Tensid-Zusammensetzungen obligatorisch. Sie haben die oben angegebene Formel (I)

R¹CH(SO₃M¹)COOM² (I),

worin der Rest R¹ einen linearen oder verzweigten Alkyl- oder Alkenylrest mit 6 bis 16 C-Atomen bedeutet und die Reste M¹ und M² - unabhängig voneinander - ausgewählt werden aus der Gruppe H, Li, Na, K, Ca/2, Mg/2, Ammonium und Alkanolamine. In Bezug auf die Verbindungen (A) gilt ferner die Maßgabe, dass der Anteil der Verbindungen (A), bei denen der Rest R¹ ein Alkyl- oder Alkenylrest mit 14 oder mehr C-Atomen ist, - bezogen auf die Gesamtmenge der Verbindungen (A) in den wäßrigen Tensid-Zusammensetzungen - bei 20 Gew.-% oder weniger liegt

In einer Ausführungsform gilt die Maßgabe, dass der Anteil der Verbindungen (A) in den wäßrigen Tensid-Zusammensetzungen, bei denen der Rest R¹ ein Alkenylrest ist, - bezogen auf die Gesamtmenge der Verbindungen (A) - bei 3 Gew.-% oder weniger liegt.

In einer bevorzugten Ausführungsform bedeutet der Rest R¹ in der Formel (I) einen gesättigten, linearen Rest mit 10 bis 16 C-Atomen, wobei in Bezug auf die Verbindungen (A) gilt, dass der Anteil der Verbindungen (A), bei denen der Rest R¹ ein Decyl- und/oder ein Dodecylrest ist , - bezogen auf die Gesamtmenge der Verbindungen (A) - bei 90 Gew.-% oder mehr liegt.

Vorzugsweise bedeuten die Reste M¹ und M² in der Formel (I) Na.

Die Verbindungen (A) können nach allen dem Fachmann einschlägig bekannten Methoden hergestellt werden. Eine besonders bevorzugte Methode der Herstellung ist dabei die Sulfierung der entsprechenden Carbonsäuren. Dabei setzt man die entsprechenden Carbonsäure und insbesondere die entsprechenden Fettsäuren mit gasförmigem Schwefeltrioxid um, wobei man das Schwefeltrioxid vorzugsweise in einer Menge einsetzt, dass das molare Verhältnis von SO₃ zu Fettsäure im Bereich von 1,0 : 1 bis 1,1 : 1 liegt. Die so erhaltenen Rohprodukte, die saure Sulfierprodukte darstellen, werden anschließend partiell oder vollständig neutralisiert, wobei eine vollständige Neutralisation mit wäßriger NaOH bevorzugt ist. Gewünschtenfalls können auch Reinigungsschritte und/oder eine Bleiche (zur Einstellung der gewünschten hellen Farbe der Produkte) vorgenommen werden.

In einer besonders bevorzugten Ausführungsform werden die Verbindungen (A) in technischer Form eingesetzt. Dies bedeutet, dass man die entsprechenden Carbonsäuren, insbesondere native Fettsäure, mit gasförmigem Schwefeltrioxid sulfiert, wodurch nach partieller oder vollständiger Neutralisation der entstehenden sauren Sulfierprodukte ein Gemisch der Verbindungen (A), (C) und (D) resultiert. Durch entsprechende Einstellungen der Reaktionsparameter (insbesondere Mol-Verhältnis von Carbonsäure und Schwefeltrioxid sowie Reaktionstemperatur) lässt sich das Verhältnis der Verbindungen (A), (C) und (D) steuern. Die Verbindungen (C) und (D) sind unten im Kapitel "Bevorzugte Ausführungsformen" beschrieben.

Im Rahmen der vorliegenden Erfindung sind solche technischen Mischungen der alpha-Sulfofettsäuredisalze bevorzugt, die wie folgt zusammengesetzt sind:
- Der Gehalt an (A) liegt im Bereich von 60 bis 100 Gew.%,
- der Gehalt an (C) liegt im Bereich von 0 bis 20 Gew.%,
- der Gehalt an (D) liegt im Bereich von 0 bis 20 Gew.%,
mit der Maßgabe, dass die Summe der Komponenten (A), (C) und (D) in dieser Mischung 100 Gew.% beträgt.

Ganz besonders sind solche technischen Mischungen bevorzugt, bei wie folgt zusammengesetzt sind:
- Der Gehalt an (A) liegt im Bereich von 70 bis 80 Gew.%,
- der Gehalt an (C) liegt im Bereich von 10 bis 15 Gew.%,
- der Gehalt an (D) liegt im Bereich von 10 bis 15 Gew.%,
mit der Maßgabe, dass die Summe der Komponenten (A), (C) und (D) in dieser Mischung 100 Gew.% beträgt.

### Zu den Verbindungen (B)

Die Verbindungen (B), die im Rahmen der vorliegenden Erfindung als Alkylglycoside bezeichnet werden, sind für die erfindungsgemäßen wäßrigen Tensid-Zusammensetzungen obligatorisch. Sie haben die oben angegebene Formel (II),

R²O[G]ₚ (I)

in der R² für einen Alkyl- und/oder Alkenylrest mit 8 bis 18 C-Atomen, G für einen Zuckerrest mit 5 oder 6 Kohlenstoffatomen und p für Zahlen zwischen 1 und 10 steht. In Bezug auf die Verbindungen (B) gilt ferner die Maßgabe, dass der Anteil der Verbindungen (B), bei denen der Rest R² ein Alkyl- oder Alkenylrest mit 15 oder mehr C-Atomen ist, - bezogen auf die Gesamtmenge der Verbindungen (B) in den wäßrigen Tensid-Zusammensetzungen - bei 5 Gew.-% oder weniger liegt.

Ausdrücklich sei festgestellt, dass die Bezeichnung der Verbindungen (B) als Alkylglykoside - fortan auch als APGs (Singular: APG) bezeichnet - lediglich einer sprachlich einfachen Bezeichnung der Verbindungen (B) dient und nicht strukturell einschränkend zu verstehen ist; denn in der formelmäßigen Definition der Verbindungen (B) ist klargestellt, dass der Rest R² sowohl einen Alkyl- als auch einen Alkenylrest bedeuten kann und ferner - dies zeigt der Index p - dass es sich um Alkyl- bzw. Alkenyloligoglykoside handelt.

APGs der hier beanspruchten Form können nach den einschlägigen Verfahren der präparativen organischen Chemie erhalten werden. Die APGs können sich von Aldosen bzw. Ketosen mit 5 oder 6 C-Atomen ableiten. Vorzugsweise leiten sich die APGs von Glucose ab.

Die Indexzahl p in der allgemeinen Formel (I) gibt den Oligomerisierungsgrad (DP-Grad = degree of polymerization) an. Der Oligomerisierungsgrad der APGs liegt zwischen 1 und 10 und vorzugsweise zwischen 1 und 6. Während p in einem einzelnen APG-Molekül stets ganzzahlig ist und hier vor allem die Werte im Bereich von 1 bis 6 annimmt, ist der Wert p für ein APG, das eine Mischung verschiedener APG-Moleküle darstellt, die sich in den je individuellen p-Werten unterscheiden, eine analytisch ermittelte rechnerische Größe, die meistens eine gebrochene Zahl darstellt. Vorzugsweise werden APGs mit einem mittleren Oligomerisierungsgrad p im Bereich von 1,1 bis 3,0 eingesetzt. Dabei sind insbesondere solche APGs bevorzugt, deren mittlerer Oligomerisierungsgrad kleiner als 2 ist und vorzugsweise im Bereich von 1,1 bis 1,8 und insbesondere im Bereich von 1,2 bis 1,7 liegt.

Der mittlere Oligomerisationsgrad ist dabei in dem Sinne zu verstehen, wie er in der Monographie K. Hill, W. von Rybinski, G. Stoll "Alkyl Polyglycosides. Technology, Properties and Applications" (VCH-Verlagsgesellschft, 1996) im Abschnitt "Degree of polymerization" (vergleiche die Seiten 11-12 des Buches) definiert ist: Dort heißt es "The average number of glycose units linked to an alcohol group is described as the (average) degree of polymerization (DP)." In der erläuternden Figur 2, die eine typische Verteilung von Dodecylglycosid Oligomeren eines AOPGs mit einem DP-Grad von 1,3 beschreibt, ist der mittlere DP-Grad auch durch eine entsprechende mathematische Formel beschrieben.

Der Rest R² leitet sich vorzugsweise von primären Alkoholen mit 4 bis 11 C-Atomen und vorzugsweise 8 bis 10 C-Atomen ab. Typische Beispiele für geeignete Reste R² sind Butyl-, Hexyl-, Octyl-, Decyl-, Undecyl-, Dodecyl- und Myristyl-. Sie leiten sich von den gesättigten Fettalkoholen Butanol-1, Capronalkohol (Hexanol-1), Caprylalkohol (Octanol-1), Caprinalkohol (Decanol-1), Undecanol-1, Laurylalkohol (Dodecanol-1) und Myristylalkohol (Tetradecanol-1) ab, wie sie beispielsweise bei der Hydrierung von technischen Fettsäuremethylestern oder im Verlauf der Hydrierung von Aldehyden bei der Roelen'schen Oxosynthese erhalten werden.

Bevorzugt sind APGs, die sich von Glucose ableiten und bei denen der Rest R² ein gesättigter Alkylrest mit 8 bis 12 C-Atomen ist und die einen mittleren Oligomerisierungsgrad im Bereich von 1,1 bis 3 und insbesondere im Bereich von 1,2 bis 1,8 und besonders bevorzugt im Bereich von 1,2 bis 1,7 aufweisen. Diese APGs können beispielsweise dadurch hergestellt werden, dass man einen Zucker, insbesondere Glucose, säurekatalysiert mit einem Fettalkoholgemisch umsetzt, wobei man als Fettsäuregemisch vorzugsweise einen bei der destillativen Auftrennung von technischem C₈₋₁₈-Kokosfettalkohol anfallenden Vorlauf einsetzt, der überwiegend Octanol-1 und Decanol-1 sowie geringe Mengen Dodecanol-1 enthält.

### Bevorzugte Ausführungsformen

In einer Ausführungsform enthalten die erfindungsgemäßen wäßrigen Tensid-Zusammensetzungen neben den Verbindungen (A), (B) und Wasser zusätzlich ein oder mehrere **Verbindungen** (C) der allgemeinen Formel (III)

R⁴COOM³ (III)

In der Formel (III) bedeutet der Rest R⁴ einen linearen oder verzweigten Alkyl- oder Alkenylrest mit 7 bis 17 C-Atomen und der Reste M³ wird ausgewählt aus der Gruppe H, Li, Na, K, Ca/2, Mg/2, Ammonium und Alkanolamine.

In einer Ausführungsform enthalten die erfindungsgemäßen wäßrigen Tensid-Zusammensetzungen neben den Verbindungen (A), (B) und Wasser zusätzlich ein oder mehrere **anorganische Salze der Schwefelsäure (D)** der allgemeinen Formel (IV)

(M⁴₎₂SO₄ (IV)

wobei M⁴ ausgewählt wird aus der Gruppe Li, Na, K, Ca/2, Mg/2, Ammonium und Alkanolamin.

Bei den Resten M¹ und M² der Verbindungen (A), dem Rest M³ der Verbindungen (C) und dem Rest M⁴ der Verbindungen (D) kann es sich um Alkanolamine handeln. Besonders bevorzugt sind dabei Monoethanolamin, Diethanolamin, Triethanolamin und Mono-Isopropanolamin.

In einer bevorzugten Ausführungsform enthalten die erfindungsgemäßen wäßrigen Tensid-Zusammensetzungen die Verbindungen (A), (B), (C) und (D). Dabei ist es besonders bevorzugt, wenn M¹ und M² der Verbindungen (A), der Rest M³ der Verbindungen (C) und der Rest M⁴ der Verbindungen (D) die Bedeutung Na (Natrium) hat.

In einer Ausführungsform liegt der Gehalt der Zusammensetzungen an den Verbindungen (A) und (B) - bezogen auf die gesamte Zusammensetzung - bei mindestens 1 Gew.-%. Vorzugsweise liegt der Gehalt der Zusammensetzungen an den Verbindungen (A) und (B) - bezogen auf die gesamte Zusammensetzung - im Bereich von 5 bis 50 Gew.-% , insbesondere im Bereich von 5 bis 20 Gew.-% und besonders bevorzugt im Bereich von 8 bis 12 Gew.-%.

Wie oben ausgeführt liegt das Gewichtsverhältnis der Verbindungen (A) : (B) in den wäßrigen Tensid-Zusammensetzungen im Bereich von 0,6 : 1 bis 1 : 0,6. Vorzugsweise liegt das Gewichtsverhältnis der Verbindungen (A) : (B) in den wäßrigen Tensid-Zusammensetzungen im Bereich von 0,7 : 1 bis 1 : 0,7 und insbesondere im Bereich von 0,8 : 1 bis 1 : 0,8. Der Bereich von 0,9 : 1 bis 1 : 0,9 ist ganz besonders bevorzugt.

In einer Ausführungsform liegt der pH-Wert der Zusammensetzungen im Bereich von 4,3 bis 5,8.

Die Viskosität der wäßrigen Tensid-Zusammensetzungen - gemessen mit einem Brookfield RV Laborrheometer bei 20°C, 12 U/min, Spindelset RV 02 bis 07 (Spindelauswahl je nach Viskositätsbereich) - liegt vorzugsweise bei 1000 mPas oder höher.

Gewünschtenfalls können die erfindungsgemäßen wäßrigen Tensid-Zusammensetzungen zusätlich ein oder mehrere weitere Tenside enthalten, die strukturell nicht zu den oben genannten Verbindungehn (A), (B), (D) oder (E) zählen. Bei diesen Tensiden kann es sich um anionische, kationische, nichtionische oder amphotere Tenside handeln.

### Verwendung der Zusammensetzungen

Ein weiterer Erfindungsgegenstand ist die Verwendung der oben genannten Zusammensetzungen für kosmetische Mittel.

Im Hinblick auf kosmetische Mittel sind dabei insbesondere solche besonders bevorzugt, die in Form von Haarshampoos, Duschgelen, Seifen, Syndets, Waschpasten, Waschlotionen, Scrub-Präparate, Schaumbädern, Ölbädern, Duschbädern, Rasierschäumen, Rasierlotionen Rasiercremes und Zahnpflegeprodukten (etwa Zahnpasten, Mundwässern und dergleichen) vorliegen.

### Beispiele

### Eingesetzte Substanzen

**SFA-I:** alpha-Sulfofettsäuredisalz technischer Qualität auf Basis von nativer C_{12/14}-Fettsäure; Zusammensetzung: 74 Gew.% Dinatrium-2-Sulfolaurat, 13 Gew.% Natrium-Laurat, 11 Gew.% Natriumsulfat, 2 Gew.-% Wasser. Die Bezeichnung "Laurat" bedeutet hierbei, dass das C12/14-Verhältnis der Mischung der zu Grunde liegenden nativen Fettsäuren 70 : 30 beträgt. Aktivsubstanzgehalt - bezogen auf Disalz - 74 Gew.-%

**APG-I:** Handelsübliches C_{12-C16}-Fettalkoholglycosid, Aktivsubstanzgehalt: 50 - 53 Gew.-%, Wasseranteil: 47-50 Gew.-% , pH-Wert ca. 11.5 - 12.5 (Planatacare 1200 UP, Fa. BASF BPCN)

### Mess- und Prüfmethoden

### Bestimmung des Anschäumverhaltens:

Zur Prüfung des Anschäumverhaltens (Rotorschaum-Methode) wurde ein handelsübliches Messgerät eingesetzt (Sita Foam Tester R-2000). Dabei wurde zunächst eine wäßrige Tensidlösung wie folgt hergestellt: 1 g Aktivsubstanz der jeweils zu prüfenden Probe (als Proben wurden SFA-I oder APG-I oder Mischungen dieser Substanzen eingesetzt, siehe unten; beim SFA-I wird - wie oben angegeben - unter Aktivsubstanzgehalt der Disalzgehalt verstanden) wurde bei 20 °C in 1 Liter Wasser mit einem Härtegrad von 15°dH (entspricht 2,673 mmol CaCO₃) gelöst. Der pH-Wert der Lösung wurde mit HCl auf einen Wert von 5,5 eingestellt. Die so hergestellt Lösung wurde temperiert auf 30°C.

Messung: Aus der temperierten Vorlage wurden 250ml in das Messgerät überführt und bei einer Umdrehungszahl von 1300 Umdrehungen pro Minute wurde für 10 Sekunden aufgeschäumt, das dann vorliegende Schaumvolumen (in ml) ermittelt, dann weitere 10 Sekunden geschäumt, das dann vorliegende Schaumvolumen (in ml) ermittelt, usw., d.h. nach jeweils 10 Sekunden währendem Aufschäumen wurde die Schaumhöhe bestimmt. Nach 80 Sekunden Aufschäumzeit wurde die Messung beendet. Die Messung wurde bei jeder Probe 3-mal mit jeweils frischer Lösung aus dem gleichen Ansatz wiederholt und das Ergebnis der Messungen nach 40, 60 und 80 Sekunden als Mittelwert aus diesen drei Messungen angegeben (siehe Tabelle).

### Beispiele

### Beispiel 1:

Es wurde eine Mischung von SFA-I und AGP-I eingesetzt, wobei das Gewichtsverhältnis der jeweiligen Aktivsubstanz von SFA-I und AGP-I auf einen Wert von 1 : 1 eingestellt war. Die Versuchsdurchführung erfolgte wie oben unter "Bestimmung des Anschäumverhaltens" beschrieben. Die Versuchsdaten können Tabelle 1 entnommen werden.

### Vergleichsbeispiel 1:

Es wurde ausschließlich SFA-I eingesetzt. Die Versuchsdurchführung erfolgte wie oben unter "Bestimmung des Anschäumverhaltens" beschrieben. Die Versuchsdaten können Tabelle 1 entnommen werden.

### Vergleichsbeispiel 2:

Es wurde ausschließlich APG-I eingesetzt. Die Versuchsdurchführung erfolgte wie oben unter "Bestimmung des Anschäumverhaltens" beschrieben. Die Versuchsdaten können Tabelle 1 entnommen werden

### Vergleichsbeispiel 3:

Es wurde eine Mischung von SFA-I und AGP-I eingesetzt, wobei das Gewichtsverhältnis der jeweiligen Aktivsubstanz von SFA-I und AGP-I auf einen Wert von 3 : 1 eingestellt war. Die Versuchsdurchführung erfolgte wie oben unter "Bestimmung des Anschäumverhaltens" beschrieben. Die Versuchsdaten können Tabelle 1 entnommen werden.

### Vergleichsbeispiel 4:

Es wurde eine Mischung von SFA-I und AGP-I eingesetzt, wobei das Gewichtsverhältnis der jeweiligen Aktivsubstanz von SFA-I und AGP-I auf einen Wert von 1 : 3 eingestellt war. Die Versuchsdurchführung erfolgte wie oben unter "Bestimmung des Anschäumverhaltens" beschrieben. Die Versuchsdaten können Tabelle 1 entnommen werden.

**Tabelle 1: Bestimmung des Anschäumverhaltens**

| | Beispiel 1 | Vergleichsbeispiel 1 | Vergleichsbeispiel 2 | Vergleichsbeispiel 3 | Vergleichsbeispiel 3 |
|---|---|---|---|---|---|
| SFA-I | 0,67 g | 1,35g | | 1,01 g | 0,34 g |
| APG-I | 0,96 g | | 1,92 g | 0,48 g | 1,44 g |
| Verhältnis SFA-I : APG-I | 1:1 | 1:0 | 0:1 | 3:1 | 1:3 |
| Schaumvolumen nach 40 sec | 430 ml | 148 ml | 333 ml | 278 ml | 173 ml |
| Schaumvolumen nach 60 sec | 618 ml | 188 ml | 433 ml | 358 ml | 235 ml |
| Schaumvolumen nach 80 sec | 868 ml | 215 ml | 519 ml | 446 ml | 292 ml |

Aus der Tabelle ist zu entnehmen, dass mit den erfindungsgemäßen Mischungen (demonstriert durch Beispiel 1) ein ganz ausgezeichnetes Schaumverhalten erzielt wird. Der Vergleich mit den Vergleichsbeispielen 1 und 2, bei denen jeweils nur Einzeltenside, also nur SFA-I oder nur APG-I eingesetzt wurden, lehrt, dass überraschenderweise ein Synergismus des schaumverhaltens vorliegt. Die Vergleichsbeispiele 3 und 4 zeigen, dass außerhalb des erfindungsgemäßen Bereiches die Mischung der Tenside SFA-I und SFA-II schlechtes Schaumverhalten zeigt.

## Patentansprüche

1. Wäßrige Tensid-Zusammensetzungen enthaltend
• ein oder mehrere alpha-Sulfofettsäuredisalze (A) der allgemeinen Formel (I),
R¹CH(SO₃M¹)COOM² (I)
worin der Rest R¹ einen linearen oder verzweigten Alkyl- oder Alkenylrest mit 6 bis 16 C-Atomen bedeutet und die Reste M¹ und M² - unabhängig voneinander - ausgewählt werden aus der Gruppe H, Li, Na, K, Ca/2, Mg/2, Ammonium und Alkanolamin,
• ein oder mehrere Alkylglycoside (B) der allgemeinen Formel (II),
R²O-[G]ₚ (I)
in der R² für einen Alkyl- und/oder Alkenylrest mit 8 bis 18 C-Atomen, G für einen Zuckerrest mit 5 oder 6 Kohlenstoffatomen und p für Zahlen zwischen 1 und 10 steht,
• Wasser,
wobei folgende Maßgaben gelten:
• In Bezug auf die Verbindungen (A) gilt, dass der Anteil der Verbindungen (A), bei denen der Rest R¹ ein Alkyl- oder Alkenylrest mit 14 oder mehr C-Atomen ist, - bezogen auf die Gesamtmenge der Verbindungen (A) in den wäßrigen Tensid-Zusammensetzungen - bei 20 Gew.-% oder weniger liegt;
• in Bezug auf die Verbindungen (B) gilt, dass der Anteil der Verbindungen (B), bei denen der Rest R² ein Alkyl- oder Alkenylrest mit 15 oder mehr C-Atomen ist, - bezogen auf die Gesamtmenge der Verbindungen (B) in den wäßrigen Tensid-Zusammensetzungen - bei 5 Gew.-% oder weniger liegt;
• sofern die wäßrigen Tensid-Zusammensetzungen ein oder mehrere Estersulfonate (E) der allgemeinen Formel (V),
R⁵CH(SO₃M⁵)COOR⁶ (V)
worin der Rest R⁵ einen linearen oder verzweigten Alkyl- oder Alkenylrest mit 6 bis 18 C-Atomen bedeutet und der Rest R⁶ einen linearen oder verzweigten Alkyl- oder Alkenylrest mit 1 bis 20 C-Atomen bedeutet, wobei der Rest R⁶ logischerweise erst ab 3 C-Atomen ein Alkenylrest sein oder verzweigt sein kann, und der Rest M⁵ ausgewählt wird aus der Gruppe Li, Na, K, Ca/2, Mg/2, Ammonium und Alkanolamine, enthält, gilt, dass die Verbindungen (A) - bezogen auf die Gesamtheit der Verbindungen (A) und (E) - zu 50 Gew.-% oder mehr vorliegen müssen;
• das Gewichtsverhältnis der Verbindungen (A) : (B) in den wäßrigen Tensid-Zusammensetzungen liegt im Bereich von 0,6 : 1 bis 1 : 0,6.

2. Zusammensetzungen nach Anspruch 1, wobei die Reste M¹ und M² Na bedeuten.

3. Zusammensetzungen nach Anspruch 1 oder 2, wobei die Zusammensetzungen zusätzlich ein oder mehrere Verbindungen (C) der allgemeinen Formel (III)
R⁴COOM³ (III)
enthält, worin der Rest R⁴ einen linearen oder verzweigten Alkyl- oder Alkenylrest mit 7 bis 19 C-Atomen bedeutet und der Reste M³ ausgewählt wird aus der Gruppe H, Li, Na, K, Ca/2, Mg/2, Ammonium und Alkanolamin.

4. Zusammensetzungen nach einem der Ansprüche 1 bis 3, wobei die Zusammensetzungen zusätzlich ein oder mehrere anorganische Salze der Schwefelsäure (D) der allgemeinen Formel (IV)
(M⁴)₂SO₄ (IV)
Enthält, wobei M⁴ ausgewählt wird aus der Gruppe Li, Na, K, Ca/2, Mg/2, Ammonium und Alkanolamin.

5. Zusammensetzungen nach einem der Ansprüche 1 bis 4, wobei der pH-Wert der Zusammensetzungen im Bereich von 4,3 bis 5,8 liegt.

6. Verwendung der Zusammensetzungen nach einem der Ansprüche 1 bis 5 für kosmetische Mittel.

7. Verwendung der Zusammensetzungen nach Anspruch 6 für kosmetische Mittel in Form von Haarshampoos, Duschgelen, Seifen, Syndets, Waschpasten, Waschlotionen, Scrub-Präparate, Schaumbädern, Ölbädern, Duschbädern, Rasierschäumen, Rasierlotionen und Rasiercremes.

## Claims

1. An aqueous surfactant composition comprising
• one or more alpha-sulfo fatty acid disalts (A) of the general formula (I),
R¹CH(SO₃M¹)COOM² (I)
in which the radical R¹ is a linear or branched alkyl or alkenyl radical with 6 to 16 carbon atoms and the radicals M¹ and M² - independently of one another - are selected from the group H, Li, Na, K, Ca/2, Mg/2, ammonium and alkanolamine,
• one or more alkyl glycosides (B) of the general formula (II),
R²O-[G]ₚ (I)
in which R² is an alkyl and/or alkenyl radical with 8 to 18 carbon atoms, G is a sugar radical with 5 or 6 carbon atoms and p is numbers between 1 and 10,
• water,
where the following provisos apply:
• with regard to the compounds (A), it is the case that the fraction of the compounds (A) in which the radical R¹ is an alkyl or alkenyl radical with 14 or more carbon atoms - based on the total amount of the compounds (A) in the aqueous surfactant composition - is 20% by weight or less;
• with regard to the compounds (B), it is the case that the fraction of the compounds (B) in which the radical R² is an alkyl or alkenyl radical with 15 or more carbon atoms - based on the total amount of the compounds (B) in the aqueous surfactant composition - is 5% by weight or less;
• if the aqueous surfactant composition comprises one or more ester sulfonates (E) of the general formula V),
R⁵CH(SO₃M⁵)COOR⁶ (V)
in which the radical R⁵ is a linear or branched alkyl or alkenyl radical with 6 to 18 carbon atoms and the radical R⁶ is a linear or branched alkyl or alkenyl radical with 1 to 20 carbon atoms, where the radical R⁶ can logically be an alkenyl radical or be branched only above 3 carbon atoms, and the radical M⁵ is selected from the group Li, Na, K, Ca/2, Mg/2, ammonium and alkanolamines, it is the case that the compounds (A) - based on the totality of the compounds (A) and (E) - must be present to 50% by weight or more;
• the weight ratio of the compounds (A) : (B) in the aqueous surfactant composition is in the range from 0.6: 1 to 1: 0.6.

2. The composition according to claim 1, where the radicals M¹ and M² are Na.

3. The composition according to claim 1 or 2, wherein the composition additionally comprises one or more compounds (C) of the general formula (III)
R⁴COOM³ (III)
in which the radical R⁴ is a linear or branched alkyl or alkenyl radical with 7 to 19 carbon atoms and the radicals M³ is selected from the group H, Li, Na, K, Ca/2, Mg/2, ammonium and alkanolamine.

4. The composition according to any one of claims 1 to 3, wherein the composition additionally comprises one or more inorganic salts of sulfuric acid (D) of the general formula (IV)
(M⁴)₂SO₄ (IV)
wherein M⁴ is selected from the group Li, Na, K, Ca/2, Mg/2, ammonium and alkanolamine.

5. The composition according to any one of claims 1 to 4, wherein the pH of the composition is in the range from 4.3 to 5.8.

6. The use of the composition according to any one of claims 1 to 5 for cosmetic products.

7. The use of the composition according to claim 6 for cosmetic products in the form of hair shampoos, shower gels, soaps, syndets, washing pastes, washing lotions, scrub preparations, foam baths, oil baths, shower baths, shaving foams, shaving lotions and shaving creams.

## Revendications

1. Compositions aqueuses de tensioactifs, contenant :
- un ou plusieurs disels d'acides alpha-sulfogras (A) de formule générale (I)
R¹CH(SO₃M¹)COOM² (I)
dans laquelle le radical R¹ signifie un radical alkyle ou alcényle linéaire ou ramifié de 6 à 16 atomes C et les radicaux M¹ et M² sont choisis indépendamment l'un de l'autre dans le groupe constitué par H, Li, Na, K, Ca/2, Mg/2, ammonium et alcanolamine,
- un ou plusieurs alkylglycosides (B) de formule générale (II)
R²O-[G]ₚ (II)
dans laquelle R² représente un radical alkyle et/ou alcényle de 8 à 18 atomes C, G représente un radical de sucre à 5 ou 6 atomes de carbone et p représente des nombres compris entre 1 et 10,
- de l'eau,
les conditions suivantes étant respectées :
- en ce qui concerne les composés (A), la proportion des composés (A) dans lesquels le radical R¹ est un radical alkyle ou alcényle de 14 atomes C ou plus, par rapport à la quantité totale des composés (A) dans les compostions aqueuses de tensioactifs, est de 20 % en poids ou moins ;
- en ce qui concerne les composés (B), la proportion des composés (B) dans lesquels le radical R² est un radical alkyle ou alcényle de 15 atomes C ou plus, par rapport à la quantité totale des composés (B) dans les compostions aqueuses de tensioactifs, est de 5 % en poids ou moins ;
- si les compositions aqueuses de tensioactifs contiennent un ou plusieurs ester-sulfonates (E) de formule générale (V)
R⁵CH(SO₃M⁵)COOR⁶ (V)
dans laquelle le radical R⁵ signifie un radical alkyle ou alcényle linéaire ou ramifié de 6 à 18 atomes C et le radical R⁶ signifie un radical alkyle ou alcényle linéaire ou ramifié de 1 à 20 atomes C, le radical R⁶ ne pouvant logiquement n'être un radical alcényle ou n'être ramifié qu'à partir de 3 atomes C, et le radical M⁵ est choisi dans le groupe constitué par Li, Na, K, Ca/2, Mg/2, ammonium et alcanolamine, les composés (A) doivent être présents, par rapport à la totalité des composés (A) et (E), à hauteur de 50 % en poids ou plus ;
- le rapport en poids des composés (A) : (B) dans les compositions aqueuses de tensioactifs se situe dans la plage allant de 0,6:1 à 1:0,6.

2. Compositions selon la revendication 1, dans lesquelles les radicaux M¹ et M² signifient Na.

3. Compositions selon la revendication 1 ou 2, dans lesquelles les compositions contiennent en outre un ou plusieurs composés (C) de formule générale (III)
R⁴COOM³ (III)
dans laquelle le radical R⁴ signifie un radical alkyle ou alcényle linéaire ou ramifié de 7 à 19 atomes C et le radical M³ est choisi dans le groupe constitué par H, Li, Na, K, Ca/2, Mg/2, ammonium et alcanolamine.

4. Compositions selon la revendication 1 à 3, dans lesquelles les compositions contiennent en outre un ou plusieurs sels inorganiques de l'acide sulfurique (D) de formule générale (IV)
(M4)₂SO₄ (IV)
dans laquelle M⁴ est choisi dans le groupe constitué par Li, Na, K, Ca/2, Mg/2, ammonium et alcanolamine.

5. Compositions selon l'une quelconque des revendications 1 à 4, dans laquelle le pH des compositions se situe dans la plage allant de 4,3 à 5,8.

6. Utilisation des compositions selon l'une quelconque des revendications 1 à 5 pour des agents cosmétiques.

7. Utilisation des compositions selon la revendication 6 pour des agents cosmétiques sous la forme de shampoings pour les cheveux, de gels douche, de savons, de détersifs synthétiques, de pâtes lavantes, de lotions lavantes, de préparations de gommage, de bains moussants, d'huiles pour le bain, de bains-douches, de mousses à raser, de lotions à raser et de crèmes à raser.
